# EUROPÄISCHE PATENTANMELDUNG

(11) **EP 4 792 755 A2**
(43) Veröffentlichungstag der Anmeldung: **19.08.2026**
(21) Anmeldenummer: 26158336.3
(22) Anmeldetag: 12.02.2026
(51) Int. Cl.: A61B 17/132

(54) **TAKTISCHER DRUCKVERBAND**

(30) Priorität: 13.02.2025 PL 45122225
(71) Anmelder: Politechnika Lodzka, 90-924 Lodz (PL); Tricomed Spolka Akcyjna, 93-493 Lodz (PL); Bella Spolka z ograniczona odpowiedzialnoscia, 87-100 Torun (PL); Wojskowy Instytut Higieny i Epidemiologii im. Gen. Karola Kaczkowskiego, 01-163 Warszawa (PL)
(72) Erfinder: Kolesiska, Beata, 95-575 Lodz (PL); Draczynski, Zbigniew, 95-073 Ustronie (PL); Skrzetuska, Ewa, 90-365 Lodz (PL); Szparaga, Grzegorz, 95-200 Pabianice (PL); Paul, Paulina, 93-173 Lodz (PL); Latanska, Ilona, 94-104 Lodz (PL); Rosiak, Piotr, 91-863 Lodz (PL); Sujka, Witold, 95-080 Tuszyn (PL); Kostkowski, Dariusz, 87-100 Torun (PL); Brytan, Marek, 05-071 Sulejôwek (PL)
(74) Vertreter: Bischof, Oliver

(57) **Zusammenfassung**

Taktischer Druckverband weist ein gemischtes Klettband mit einem kürzeren Teil (1) und einem längeren Teil (2) auf, die jeweils eine glatte Seite und eine Seite mit Haken und Schlaufen haben. Der Druckverband umfasst auch ein Außenband (3) und ein Innenband (4), welche miteinander kontaktieren und lediglich an einigen Stellen miteinander verbunden sind,
- wobei das Außenband (3) mit der glatten Seite des kürzeren Teils (1) verbunden ist und eine Schlaufe bildet, in der eine Schnalle (5) mit einem hakenförmigen Ende angeordnet ist,
- wobei auf der Oberfläche des Innenbands (3) ein Zahnriemen (6) mit einer Ratschenklammer (7) befindlich ist.

An einem Ende des längeren Teils (2) des Klettbands ein Fingerloch (8) und eine Markierung (9) angeordnet sind.

Die Ratschenklammer (7), der Zahnriemen (6) und das Innenband (4) bilden einen Spannmechanismus, der eine Kompressionskraft erzeugt und den Blutfluss an der verletzten Extremität stoppt.

## Beschreibung

Die Erfindung betrifft einen taktischen Druckverband, der insbesondere für militärische Anwendungen bestimmt ist und dazu dient, den Blutfluss abzusperren und Blutungen an den unteren und oberen Extremitäten in Notfallsituationen zu stoppen.

Die Hauptaufgabe eines taktischen Druckverbands besteht im vollständigen Verschließen des Blutgefäßlumens in Fällen, in denen herkömmliche Methoden zur Eindämmung massiver Blutungen, wie das Hochlagern der Extremität, ein Druckverband oder eine Kompression unwirksam sind. Ein taktischer Druckverband sollte bei massiven Blutungen und bei Amputationen von Gliedmaßen verwendet werden. Durch die Erhöhung des umlaufenden Drucks wird eine Blutung wirksam gestoppt. Eine der Hauptanwendungen von taktischen Druckverbänden ist ihre Verwendung in der Ersten Hilfe auf dem Schlachtfeld. Wesentliche Anforderungen an einen taktischen Druckverband bestehen darin, dass er auf einfache Art und Weise an eine Körpergliedmaße angelegt und gleichzeitig einfach, schnell und effektiv festgezogen werden kann.

Alle oben genannten Merkmale dienen dazu, die Anwendung eines taktischen Druckverbands zu vereinfachen, die Unannehmlichkeiten während seiner Verwendung zu minimieren und Blutungen zu stoppen. Es gibt viele Lösungen, welche die oben genannten Eigenschaften eines taktischen Druckverbands gewährleisten sollen. Die auf dem Markt erhältlichen taktischen Druckverbände unterscheiden sich im Design, im Gewicht und in der Art und Weise, wie sie festgezogen und gesichert werden können, um eine versehentliche Druckentlastung zu verhindern.

Einer der am häufigsten verwendeten Ansätze zur Erzeugung von Spannung bei taktischen Druckverbänden basiert auf einem Stift (Knebel) zusammen mit einem blockierenden Element. Der Druckverband wird durch Drehen des Stifts, durch den das Gurtband des taktischen Druckverbands gezogen wird, an der Extremität festgezogen. Durch die Einwirkung auf den Knebel wird der Druckverband zusammengezogen und anschließend wird der Knebel arretiert, sodass der Druck nicht nachlässt. In taktischen Tourniquets und Druckverbänden werden auch Spulen mit Drehknöpfen, Kerben, Schlüssel, Zugseile mit Trommeln, Schrauben, Zahnradgetriebe, hakenförmige Elemente und Ratschenmechanismen verwendet. Eine weitere Lösung, die bei taktischen Druckverbänden zum Einsatz kommt, ist die Art und Weise, wie der Druckverband verbunden wird. Bei den bekannten Lösungen werden meist Klammern, aber auch Verbindungselemente, Ringe, Halterungen, Aufnahmen, Platten, Schnallen, Befestigungselemente (z. B. Haken) oder klassische Bindemethoden angewandt. Trotz ihrer guten Wirksamkeit beim Stillen von Blutungen ermöglichen nicht alle der vorgeschlagenen Verfahren auch eine einfache Druckentlastung. Ein wichtiges Merkmal von taktischen Druckverbänden sollte ihre einfache Handhabung sowie die für ihr Anlegen benötigte Zeit sein. Dies ist aufgrund des Verwendungszwecks des Druckverbands wichtig. Ein taktischer Druckverband wird bei plötzlichen Blutungen eingesetzt, bei denen eine schnelle und einfache Anwendung erforderlich ist und wo ein Verband einen vollständigen Verschluss der Blutgefäße ermöglichen sollte.

Aus der Patentschrift EP 1753344 B1 ist ein Tourniquet bekannt, umfassend einen Griff (Knebel), der durch Drehung eine Spannung um die Extremität erzeugt. Je nach Drehrichtung des Knebels kann der taktische Druckverband mehr oder weniger gut zu handhaben sein, aber in beiden Fällen verlängert sich die zum Anlegen des Produkts benötigte Zeit. Der Knebel ist in einer hakenförmigen Halterung gesichert, was das Verriegeln des Griffs einfach macht. Die Positionierung des Knebels selbst kann jedoch dazu führen, dass er sich an verschiedenen Gegenständen verfängt und somit zur Unbequemlichkeit führen kann. Das Tourniquet umfasst eine geschlossene Schnalle, die zusammen mit einem Kompressionsband Schlaufen um die betroffene Extremität bildet. Die Verwendung einer geschlossenen Schnalle wirkt sich auf die Zeit aus, die für das Anlegen des Druckverbands benötigt wird, da diese vom Tourniquet gelöst und dann wieder angebracht werden muss. Dadurch erhöht sich der Zeitaufwand für das Anlegen des Verbands an den unteren Gliedmaßen erheblich, was die Effektivität der Anwendung des in EP 1753344 B1 beschriebenen Verbands beeinträchtigen kann.

Aus der Patentanmeldung WO 2005/053506 A2 ist eine Staumanschette bekannt, die ein bogenförmiges Element aufweist, welches das Befestigen der Manschette an einem Körperteil ermöglicht, sowie eine primäre und eine sekundäre, an einem bogenförmigen Element angebrachte Spanneinheit und eine Verriegelungseinheit. Die primäre Spanneinheit umfasst ein Versorgungssystem mit mechanischem Vorteil, das eine Schnuraufrolleinheit beinhaltet, um einen mechanischen Vorteil zu bieten, wenn es vom Benutzer verwendet wird.

Diese Einheit umfasst einen drehbaren und schwenkbaren Drehschlüssel, der zum Drehen der Schnuraufrolleinheit dient. Das vorgestellte System erfordert einen zusätzlichen Schutz, der die Vorrichtung umgibt, um eine Beschädigung der Haut des Benutzers oder einen Mangel am Komfort während der Nutzung zu vermeiden. Die zusätzlich verwendeten Materialien erhöhen das Gewicht und den Preis des fertigen Produkts. Die Staumanschette enthält lumineszierende Elemente, die in dunkler Umgebung als Markierung dienen. In lebensbedrohlichen Situationen, z. B. auf dem Schlachtfeld, können sie aber auch möglicherweise das Risiko bergen, dass die Position der Person, die das Produkt trägt, entdeckt wird. Die Verriegelungseinheit umfasst eine C-förmige Klemme und einen Verschluss. Das Kompressionssystem zusammen mit der Verriegelungseinheit sind so aufgebaut, dass sie zusammen einen Spannmechanismus bilden. Ein Nachteil dieses Mechanismus besteht in der Möglichkeit einer unbeabsichtigten Druckentlastung, wenn die Manschette festgezogen wird, und ihrer Blockierung in der zweiteiligen Verriegelungseinheit.

Aus der US-Patentanmeldung US 2021/0106340 A1 ist ein Druckverband bekannt, der einen Verband, ein Windenband und einen Windengriff umfasst. Zu den Spannelementen gehört der Windengriff, der einen Windenstab beinhaltet. Das Drehen des Windenstabs mit Hilfe des Windengriffs bewirkt das Aufwickeln des Verbands und somit eine Druckausübung auf die Extremität. Es kommt zu einer Verkürzung der effektiven Länge des Windenbands. Zusätzlich weist der in der genannten Patentanmeldung beschriebene Druckverband ein Abdeckband auf, das den Windengriff und das Windenband abdeckt. Das Abdeckband ist ein einstückiges Materialstück, das in der gleichen Ebene liegt wie der oben erwähnte Windengriff. Durch das Zusatzband erhöht sich das Flächengewicht des Produkts. Außerdem enthält der Druckverband eine Aufbewahrungsklappe, mit der das Abdeckband durch Klettverschluss verbunden ist. Der Griffmechanismus weist einen Schlitz zur Aufnahme und zum Halten eines Teils des Windengriffs auf, um eine Gegendrehung unter der auf das Windenband wirkende Kraft zu verhindern. Der flache Griffmechanismus und das Verfahren zum Blockieren des Windenstabs kann sich für Benutzer in Notsituationen als unintuitiv erweisen. Die Form der Winde kann den Nutzungskomfort beim Tragen des Tourniquets beeinflussen, weil das Tourniquet dadurch an einem Teil des Bandes versteift wird. Der in der genannten Anmeldung dargestellte Druckverband hat eine gurtartige Schnalle am Schnallenende des Bandes und eine Schnalle zur Verbindung mit der Schlaufe. Der Windengriff-Mechanismus weist zwei Schlitze zur Aufnahme und zum Halten der Winde auf. Die Vielzahl der Komponenten, die zum Spannmechanismus des beschriebenen Verbands gehören, hat zur Folge, dass sich das Gewicht des Produkts erhöht und die Zeit für das Anlegen des Druckverbands verlängert.

Die Patentanmeldung WO 2015/048660 A1 beschreibt ein Tourniquet, das einen Tourniquet-Körper, einen Schlitten, einen Torsionsstab, eine Aufnahme und einen Schieber beinhaltet. Durch das Drehen des Torsionsstabs wird das Tourniquet um den Umfang der Extremität herum festgezogen, wodurch eine Kompressionskraft entsteht, die den Blutfluss in der Extremität einschränkt. Der Stab wird von einer am Basiselement befestigten Halterung gehalten. Der Nachteil eines solchen Mechanismus ist der fehlende Schutz des Torsionsstabs, der durch eine ungewollte Krafteinwirkung aus der Halterung gezogen werden kann, was zur Druckentlastung führt. Schlaufenbildung um die Gliedmaßen wird durch einen Schieber mit einer passenden Aufnahme erreicht. Durch die Kombination dieser Elemente wird eine umlaufende Kompression um die Extremität erzeugt. Diese Lösung hat zur Folge, dass das Tourniquet mit beiden Händen angelegt werden muss, was bei Verletzungen der oberen Gliedmaßen problematisch sein kann. Das in der Anmeldung beschriebene Tourniquet umfasst einen Tourniquet-Körper und einen Schlitten, der am Tourniquet-Körper befestigt und mit diesem ausgerichtet ist. Der Torsionsstab zusammen mit einem Spanngurt sind an der Außenseite des Tourniquet-Körpers und an der Außenseite des Schlittens angeordnet. Beim Drehen des Torsionsstabs wird der Spanngurt festgezogen und der Körper wird gleichmäßig in Richtung des Schlittens gezogen. Das gezeigte Verfahren verhindert nicht die Möglichkeit der Entstehung von Stofffalten, was dem Träger Unannehmlichkeiten bereiten und die Haut beim Anlegen des Tourniquets zusätzlich schädigen kann.

Aus der Patentanmeldung WO 2020/227697 ist ein Tourniquet bekannt, umfassend eine aus zwei Teilen bestehende Basis, wobei an einem dieser Teile eine Kappe angebracht ist. An der Basis ist ein Kompressionsriemen befestigt, der über die gesamte Länge mit ihr verbunden ist. Die Ausübung einer Druckkraft auf den Riemen kann dazu führen, dass das Material Falten bildet und ungleichmäßig wird. Dies hat Auswirkungen auf den Tragekomfort, denn ein unsachgemäß angelegter Verband kann die Haut beschädigen. Der Windengriff weist eine Öffnung für den Kompressionsriemen auf, durch die der Riemen gefädelt und vernäht wird, um Schlaufen um die Öffnung zu bilden. Ein Verriegelungsriemen ist mit der Kappe verbunden, wobei ein Griffanschlag in den Verriegelungsriemen so eingreift, dass ein freies Gleiten entlang des Griffriemens folgt. Die Winde hat keine Sicherung, die sicherstellen würde, dass sich das Tourniquet im Feld nicht irgendwo verfängt. Eine Riemenverbindungsanordnung umfasst einen Schnallenrahmen und einen selbst-sichernden Verschiebearm. Der sich quer erstreckende Verschiebearm ist so eingerichtet, dass er entlang des Schnallenrahmens verschiebbar ist. Der Tourniquet-Riemen wird durch den Schnallenrahmen gefädelt, wobei ein Schnallenverbinder so am Riemen befestigt ist, um in den Schnallenrahmen einzugreifen. Das Tourniquet weist zusätzlich eine Griffverriegelung auf, die mit dem Verriegelungsriemen verschiebbar verbunden ist. Der Spannmechanismus des Verbands besteht aus mehreren Komponenten, deren Aufbau sowohl die Handhabung des Produkts als auch die Zeit zum Anlegen beeinträchtigen kann.

Aus der Patentanmeldung WO 2021/155031 A1 ist ein Druckverband bekannt, umfassend eine Gewindestange, eine Druckplatte, die mit einem zweiten Stangenende verbunden ist und wenigstens eine Ratschensperre, die das Gewinde der Ratschensperre definiert. Die Gewindestange ist in einer ersten axialen Richtung und einer zweiten axialen Richtung beweglich, wobei die Druckplatte sich in Richtung von mindestens einer Ratschensperre bewegt. Die Gewindegänge der Ratschensperre sind so geformt, um einer Verschiebung der Gewindestange in einer ersten axialen Richtung, wenn sie sich in der axialen Position befindet, entgegenzuwirken, und um mindestens eine Ratschensperre aus der Eingriffsposition in die Ausrückposition zu drücken, wenn die Gewindestange in der zweiten axialen Richtung bewegt wird. Ein aus einer Gewindestange und Ratschensperren bestehender Spannmechanismus ist im Vergleich zu den verwendeten Knebeln langsamer in der Anwendung. Er bestimmt auch das Gewicht des Produkts und seine Handhabbarkeit. Die Verwendung eines solchen Druckverbands im Feld kann aufgrund des unpraktischen, hervorstehenden Knebelgriffs erschwert sein.

In den verfügbaren Quellensammlungen in Form von Patentbeschreibungen und Beschreibungen von Patentanmeldungen werden die Konstruktionen von taktischen Tourniquets vorgestellt sowie die Mechanismen zur Erreichung und Aufrechterhaltung des Drucks, dessen Aufgabe es ist, den Blutfluss in der Gliedmaße zu stoppen. Die meisten Lösungen beinhalten einen Knebel, der zur Ausübung von Druck auf die ausgewählte Extremität dient, sowie ein Blockiersystem, dessen Aufgabe es ist, den Knebel in einer festen Position zu halten. Die vorgestellten Lösungen konzentrieren sich auf das effiziente Anlegen von Druck und dessen einfache Entlastung sowie auf das Verfahren zur Bildung einer Schlaufe um die Extremität.

Es ist jedoch sinnvoll, eine Lösung zu finden, die den Komfort, die Benutzerfreundlichkeit und die Schnelligkeit der Anwendung des taktischen Druckverbands verbessert und seine angemessene Tarnung im Schlachtfeld gewährleistet.

Das Ziel der vorliegenden Erfindung ist es, einen taktischen Druckverband anzugeben, der massive Blutungen wirksam stoppt und gleichzeitig die durch das Tragen des Druckverbands verursachten Unannehmlichkeiten minimiert sowie eine einfache und schnelle Anwendung in Notfallsituationen garantiert.

Die Konstruktion des erfindungsgemäßen taktischen Druckverbands macht es möglich, die Blutgefäße zu verschließen, indem der Träger selbst den Druck mit einer Hand, nämlich mit einem Finger, reguliert.

Die Erfindung betrifft einen taktischen Druckverband, der in erster Linie für militärische Zwecke bestimmt ist.

Der taktische Druckverband gemäß Erfindung zeichnet sich durch seine einzigartige Konstruktion aus.

Der taktische Druckverband weist ein kürzeres Teil eines gemischten Klettbands und ein längeres Teil eines gemischten Klettbands auf, wobei sowohl das kürzere Teil des gemischten Klettbands als auch das längere Teil des gemischten Klettbands eine glatte Seite und eine Seite mit Haken und Schlaufen haben. Der taktische Druckverband weist darüber hinaus ein Außenband und ein Innenband auf, wobei das Außenband und das Innenband miteinander kontaktieren und lediglich über die Breite an einigen Stellen miteinander verbunden sind, wobei das Außenband mit der glatten Seite des kürzeren Teils des gemischten Klettbands verbunden ist und eine Schlaufe bildet, in der eine Schnalle angeordnet ist.

Die Schnalle stellt ein offenes Element mit einem hakenförmigen Ende dar. Auf der Oberfläche des Innenbands ist ein Zahnriemen angeordnet, an welchem wiederum eine Ratschenklammer angeordnet ist, die nicht mehr als 1 cm von der die Schnalle befestigender Schlaufe entfernt ist. Das längere Teil des gemischten Klettbands ist mit dem Außenband über eine Länge nicht kleiner als ein achtel und nicht größer als die Hälfte seiner Länge, wie auch über die Breite des Außenbands und des Innenbands verbunden, wobei am Ende des längeren Teils des gemischten Klettbands ein Fingerloch befindlich ist.

Bevorzugt ist die Länge des Innenbands kürzer als die Gesamtlänge des taktischen Druckverbands.

Die Ratschenklammer ist mit wenigstens einem Zahn des Zahnriemens verbunden.

Die Ratschenklammer, der Zahnriemen und das Innenband bilden einen Spannmechanismus, bei dem die Druckkraft durch Aufbringen einer Zugkraft an die Ratschenklammer erzielt wird, was eine Schiebebewegung des Zahnriemens und des Innenbands bewirkt und somit eine Kompressionskraft erzeugt, wobei die Kompressionskraft in Umfangsrichtung auf die jeweilige verletzte Extremität wirkt und den Blutfluss stoppt.

Das Innenband und das Außenband gehören zur Gruppe der flachen Textilerzeugnisse.

Das Innenband und das Außenband sind gewebte Textilerzeugnisse, die vorzugsweise aus Garnen aus der Gruppe der Chemiefasern bestehen.

Das Außenband kann aus Polyamid, und das Innenband aus Polyester bestehen.

Die Schnalle weist die Form der Ziffer "9" auf.

Am Ende des längeren Teils des gemischten Klettbands befindet sich eine Öffnung, die mit einer Markierung, vorzugsweise in Rot, versehen sein kann.

Das kürzere Teil des gemischten Klettbands und das längere Teil des gemischten Klettbands können mit einem Suspensionsfarbstoff gefärbt sein, der vorzugsweise aus der Gruppe von syntenischen Farbstoffen ausgewählt ist.

Die Färbelösung kann 1 bis 3 Gew.-% eines Suspensionsfarbstoffs enthalten, vorzugsweise 2 Gew.-% eines Suspensionsfarbstoffs.

Die Erfindung wird anhand der folgenden Ausführungsbeispiele und Zeichnungen näher erläutert. Die Figuren zeigen:

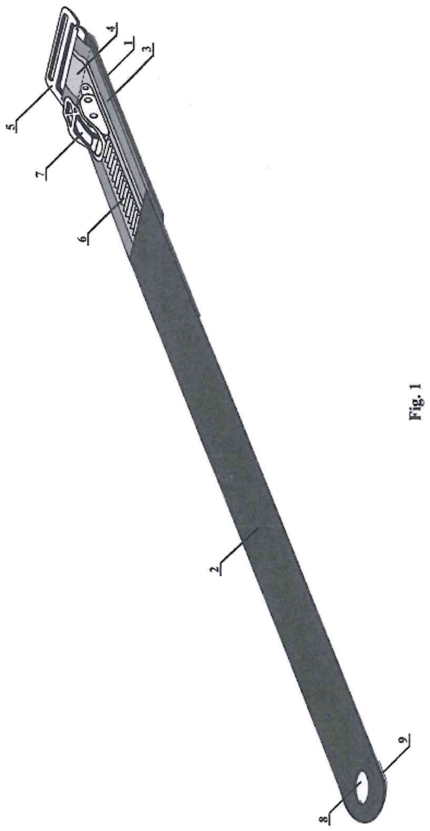

Fig.1 eine Draufsicht auf das Produkt,

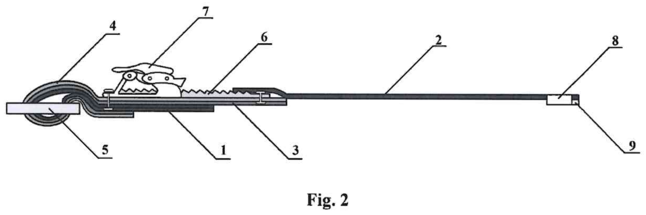

Fig. 2 das Produkt in einer perspektivischen Ansicht, in der die einzelnen Komponenten des Produkts detailliert dargestellt sind.

### Beispiel 1

Ein taktischer Druckverband weist ein kürzeres Teil 1 eines gemischten Klettbands und ein längeres Teil 2 des gemischten Klettbands auf, wobei sowohl das kürzere Teil 1 des gemischten Klettbands als auch das längere Teil 2 des gemischten Klettbands eine glatte Seite und eine Seite mit Haken und Schlaufen haben. Der taktische Druckverband weist darüber hinaus ein aus Polyamid gefertigtes Außenband 3 und ein aus Polyester gefertigtes Innenband 4, wobei beide Bänder miteinander kontaktieren, aber lediglich über die Breite an drei Stellen miteinander verbunden sind, wobei das Außenband 3 mit der glatten Seite des kürzeren Klettbands 1 verbunden ist und eine Schlaufe bildet. In der Schlaufe ist eine Schnalle 5 angeordnet, welche die Form der Ziffer "9" aufweist, wobei die Schnalle 5 ein offenes Element mit einem hakenförmigen Ende darstellt. Auf der Oberfläche des Innenbands 4 ist ein Zahnriemen 6 angeordnet, an welchem wiederum eine Ratschenklammer 7 angeordnet ist, die 1 cm von der die Schnalle 5 befestigenden Schlaufe entfernt ist. Die Ratschenklammer 7 ist mit einem Zahn des Zahnriemens 6 verbunden. Das längere Teil 2 des gemischten Klettbands ist mit dem Außenband 3 über eine Länge von einem viertel seiner Länge, wie auch über die Breite des Außenbands 3 und des Innenbands 4 verbunden. Außerdem befindet sich am Ende des längeren Teils 2 des gemischten Klettbands ein Fingerloch 8, das mit einer Markierung 9 in Rot versehen ist.

Die Ratschenklammer 7, der Zahnriemen 6 und das Innenband 4 bilden einen Spannmechanismus, bei dem die Druckkraft durch Aufbringen einer Zugkraft an die Ratschenklammer 7 erzielt wird, was eine Schiebebewegung des Zahnriemens 6 und des Innenbands 4 bewirkt und somit eine Kompressionskraft erzeugt, wobei die Kompressionskraft in Umfangsrichtung auf die jeweilige verletzte Extremität wirkt und den Blutfluss stoppt.

### Beispiel 2

Ein taktischer Druckverband weist ein kürzeres Teil 1 eines gemischten Klettbands und ein längeres Teil 2 des gemischten Klettbands auf, wobei sowohl das kürzere Teil 1 des gemischten Klettbands als auch das längere Teil 2 des gemischten Klettbands eine glatte Seite und eine Seite mit Haken und Schlaufen haben. Der taktische Druckverband weist darüber hinaus ein aus Polyamid gefertigtes Außenband 3 und ein aus Polyester gefertigtes Innenband 4 auf, wobei die Länge des Innenbands 4 kürzer als die Gesamtlänge des taktischen Druckverbands ist und wobei beide Bänder miteinander kontaktieren, aber lediglich über die Breite an fünf Stellen miteinander verbunden sind, wobei das Außenband 3 mit der glatten Seite des kürzeren Teils 1 des gemischten Klettbands verbunden ist und eine Schlaufe bildet. In der Schlaufe ist eine Schnalle 5 angeordnet, welche die Form der Ziffer "9" aufweist, wobei die Schnalle 5 ein offenes Element mit einem hakenförmigen Ende darstellt. Auf der Oberfläche des Innenbands 4 ist ein Zahnriemen 6 angeordnet, an welchem wiederum eine Ratschenklammer 7 angeordnet ist, die 0,5 cm von der die Schnalle 5 befestigender Schlaufe entfernt ist. Die Ratschenklammer 7 ist mit drei Zähnen des Zahnriemens 6 verbunden. Das längere Teil 2 des gemischten Klettbands ist mit dem Außenband 3 über eine Länge von einem fünftel seiner Länge, wie auch über die Breite des Außenbands 3 und des Innenbands 4 verbunden. Außerdem befindet sich am Ende des längeren Teils 2 des gemischten Klettbands ein Fingerloch 8, das mit einer Markierung 9 in Rot versehen ist. Gleichzeitig sind das kürzere Teil 1 des gemischten Klettbands und das längere Teil 2 des gemischten Klettbands mit einem Suspensionsfarbstoff aus der Gruppe der syntenischen Farbstoffen gefärbt, von dem 2 Gew.-% in der Färbelösung enthalten waren.

Die Ratschenklammer 7, der Zahnriemen 6 und das Innenband 4 bilden einen Spannmechanismus, bei dem die Druckkraft durch Aufbringen einer Zugkraft an die Ratschenklammer 7 erzielt wird, was eine Schiebebewegung des Zahnriemens 6 und des Innenbands 4 bewirkt und somit eine Kompressionskraft erzeugt, wobei die Kompressionskraft in Umfangsrichtung auf die jeweilige verletzte Extremität wirkt und den Blutfluss stoppt.

### Bezugszeichenliste:

- 1: kürzeres Teil
- 2: längeres Teil
- 3: Außenband
- 4: Innenband
- 5: Schnalle
- 6: Zahnriemen
- 7: Ratschenklammer
- 8: Fingerloch
- 9: Markierung

## Patentansprüche

1. Taktischer Druckverband, **dadurch gekennzeichnet, dass** er ein kürzeres Teil (1) eines gemischten Klettbands und ein längeres Teil (2) eines gemischten Klettbands aufweist, wobei sowohl das kürzere Teil (1) des gemischten Klettbands als auch das längere Teil (2) des gemischten Klettbands eine glatte Seite und eine Seite mit Haken und Schlaufen haben,
und dass er darüber hinaus ein Außenband (3) und ein Innenband (4) aufweist,
- wobei das Außenband (3) und das Innenband (4) miteinander kontaktieren und lediglich über die Breite an einigen Stellen miteinander verbunden sind,
- wobei das Außenband (3) mit der glatten Seite des kürzeren Teils (1) des gemischten Klettbands verbunden ist und eine Schlaufe bildet, in der eine Schnalle (5) angeordnet ist,
- wobei die Schnalle (5) ein offenes Element mit einem hakenförmigen Ende darstellt,
- wobei auf der Oberfläche des Innenbands (4) ein Zahnriemen (6) befindlich ist, an welchem wiederum eine Ratschenklammer (7) angeordnet ist, die nicht mehr als 1 cm von der die Schnalle (5) befestigenden Schlaufe entfernt ist,
- wobei das längere Teil (2) des gemischten Klettbands mit dem Außenband (3) über eine Länge nicht kleiner als ein achtel und nicht größer als die Hälfte seiner Länge, wie auch über die Breite des Außenbands (3) und des Innenbands (4) verbunden ist,
- wobei am Ende des längeren Teils (2) des gemischten Klettbands ein Fingerloch (8) befindlich ist.

2. Taktischer Druckverband nach Anspruch 1, **dadurch gekennzeichnet, dass** die Länge des Innenbands (4) kürzer als die Gesamtlänge des taktischen Druckverbands ist.

3. Taktischer Druckverband nach Anspruch 1 oder 2, **dadurch gekennzeichnet, dass** die Ratschenklammer (7) mit wenigstens einem Zahn des Zahnriemens (6) verbunden ist.

4. Taktischer Druckverband nach einem der vorhergehenden Ansprüche, **dadurch gekennzeichnet, dass** die Ratschenklammer (7), der Zahnriemen (6) und das Innenband (4) einen Spannmechanismus bilden, bei dem die Druckkraft durch Aufbringen einer Zugkraft an die Ratschenklammer (7) erzielt wird, was eine Schiebebewegung des Zahnriemens (6) und des Innenbands (4) bewirkt und somit eine Kompressionskraft erzeugt, wobei die Kompressionskraft in Umfangsrichtung auf die jeweilige verletzte Extremität wirkt und den Blutfluss stoppt.

5. Taktischer Druckverband nach einem der vorhergehenden Ansprüche, **dadurch gekennzeichnet, dass** das Innenband (4) und das Außenband (3) zur Gruppe der flachen Textilerzeugnisse gehören.

6. Taktischer Druckverband nach Anspruch 5, **dadurch gekennzeichnet, dass** das Innenband (4) und das Außenband (3) gewebte Textilerzeugnisse sind, die vorzugsweise aus Garnen aus der Gruppe der Chemiefasern bestehen.

7. Taktischer Druckverband nach Anspruch 6, **dadurch gekennzeichnet, dass** das Außenband (3) aus Polyamid, und das Innenband (4) aus Polyester besteht.

8. Taktischer Druckverband nach einem der vorhergehenden Ansprüche, **dadurch gekennzeichnet, dass** die Schnalle (5) die Form der Ziffer "9" aufweist.

9. Taktischer Druckverband nach einem der vorhergehenden Ansprüche, **dadurch gekennzeichnet, dass** am Ende des längeren Teils (2) des gemischten Klettbands sich ein mit einer Markierung, vorzugsweise in Rot, versehenes Fingerloch (8) befindet.

10. Taktischer Druckverband nach einem der vorhergehenden Ansprüche, **dadurch gekennzeichnet, dass** das kürzere Teil (1) des gemischten Klettbands und das längere Teil (2) des gemischten Klettbands mit einem Suspensionsfarbstoff gefärbt sind, der vorzugsweise aus der Gruppe von syntenischen Farbstoffen ausgewählt ist.

11. Taktischer Druckverband nach Anspruch 10, **dadurch gekennzeichnet, dass** die Färbelösung 1 bis 3 Gew.-% eines Suspensionsfarbstoffs enthält.

12. Taktischer Druckverband nach Anspruch 10, **dadurch gekennzeichnet, dass** die Färbelösung 2 Gew.-% eines Suspensionsfarbstoffs enthält.
